(19) Europäisches Patentamt
European Patent Office
Office européen des brevets

(11) Publication number: **0 043 263**
**B1**

(12) **EUROPEAN PATENT SPECIFICATION**

(45) Date of publication of patent specification: **25.07.84**

(21) Application number: **81302914.7**

(22) Date of filing: **26.06.81**

(51) Int. Cl.³: **C 07 D 513/04** //A01N47/24, (C07D513/04, 277/00, 235/00), (C07D513/04, 277/00, 239/00), (C07D513/04, 277/00, 243/00), (C07D513/04, 277/00, 249/00)

# (54) Bicyclic 2-iminothiazolidine oximes and methods of preparing same.

(30) Priority: **27.06.80 US 163631**

(43) Date of publication of application:
**06.01.82 Bulletin 82/1**

(45) Publication of the grant of the patent:
**25.07.84 Bulletin 84/30**

(84) Designated Contracting States:
**BE CH DE FR GB IT LI NL**

(56) References cited:
**JOURNAL OF ORGANIC CHEMISTRY, vol. 45, no. 21, 10th October 1980, pages 4198-4206, Washington, U.S.A., D.F. BUSHEY et al.: "Syntheses and stereochemistry of amidoximes"**

(73) Proprietor: **UNION CARBIDE CORPORATION**
**Old Ridgebury Road**
**Danbury Connecticut 06817 (US)**

(72) Inventor: **Bushey, Dean Franklin**
**658 Forest Circle**
**South Charleston 25303 West Virginia (US)**
Inventor: **D'Silva, Themistocles Damasceno Joaquim**
**1202 Ellen Drive**
**South Charleston 25303 West Virginia (US)**

(74) Representative: **Baverstock, Michael George Douglas et al,**
**BOULT, WADE & TENNANT 27 Furnival Street**
**London, EC4A 1PQ (GB)**

**EP 0 043 263 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to certain bicyclic 2-iminothiazolidine oximes and methods of preparing same.

More particularly, this invention relates to compounds of the formula

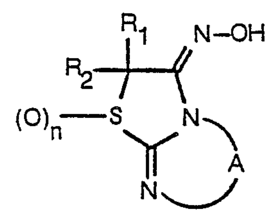

wherein $R_1$ and $R_2$ are the same or different and are individually hydrogen, $C_1$—$C_6$ alkyl or halogen;

n = 0, 1 or 2; and

A is acyclic, cyclic or bicyclic alkylene or alkenylene group, or an ortho-substituted phenylene group which forms an unsaturated five, six or seven membered heterocyclic ring which can also contain oxygen, sulfur, nitrogen, or carbonyl in addition to the two nitrogens, said alkylene, alkenylene, or phenylene ring systems being optionally substituted with one or more $C_1$—$C_4$ alkyl, cyclohexyl, alkoxy, phenoxy, alkylthio, halogen, amido, alkylamido, nitrile, acyl, or nitro groups.

Compounds falling within the above generic formula have utility as intermediates and/or precursors for the formation of compounds which exhibit biological activity as insecticides.

In general, the 2-iminothiazolidine oximes of this invention can be conveniently prepared by reacting a cyclic thiourea with the dichloro-oxime as described below, in which $R_1$, $R_2$, and A are as described previously.

METHOD I

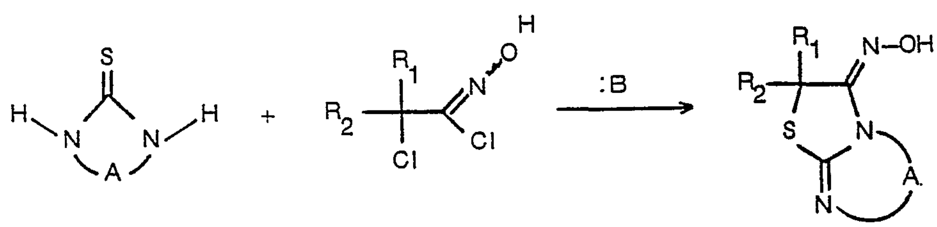


The reaction of Method I can be conducted in aqueous or organic solvents with an appropriate acid acceptor. The acid acceptor in the conduct of the reaction of Method I may be either an organic or inorganic base. Aromatic and aliphatic tertiary amines, such as triethylamine, pyridine, and 1,4-diazobicyclo[2.2.2] octane are representative of the organic bases. Bases such as sodium hydroxide, potassium carbonate and sodium bicarbonate are illustrative of the inorganic bases which are useful in the conduct of this reaction.

When an inorganic base is used phase transfer agents may be used to facilitate the transfer of the base across the organic/inorganic phase interface. Illustrative of useful phase transfer agents are crown ether compounds, and quaternary ammonium halide compounds.

The reactions are preferably carried out in methanol using sodium bicarbonate as the acid acceptor. The temperature is preferably held between −78°C and 0° during addition of starting materials and the mixture is slowly allowed to warm to 25°C. The time of reaction varies between 16 hours and 3 days.

The insecticidal compounds which utilize the oxime compounds of this invention as precursors can be prepared by a variety of methods, the methods are illustrated by the reaction schemes set forth below.

METHOD II

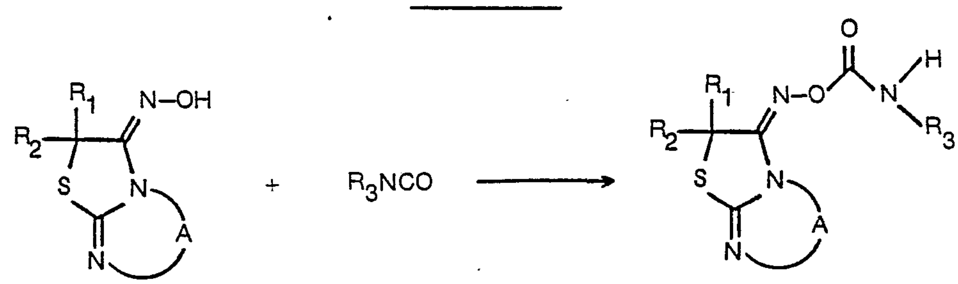

METHOD III

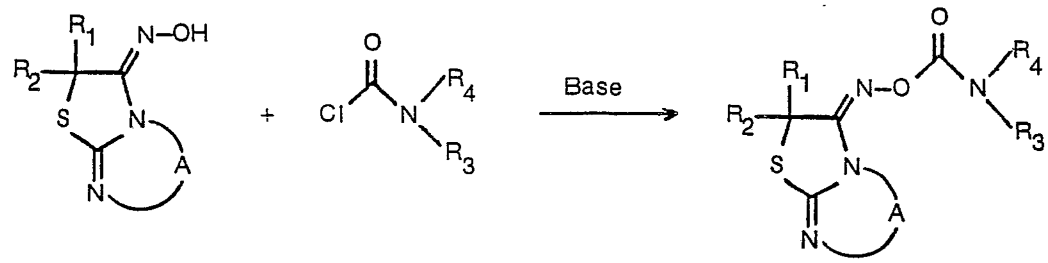


METHOD IV

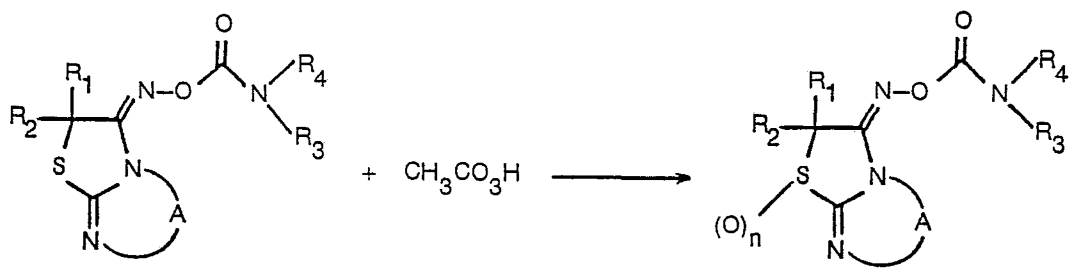

METHOD V

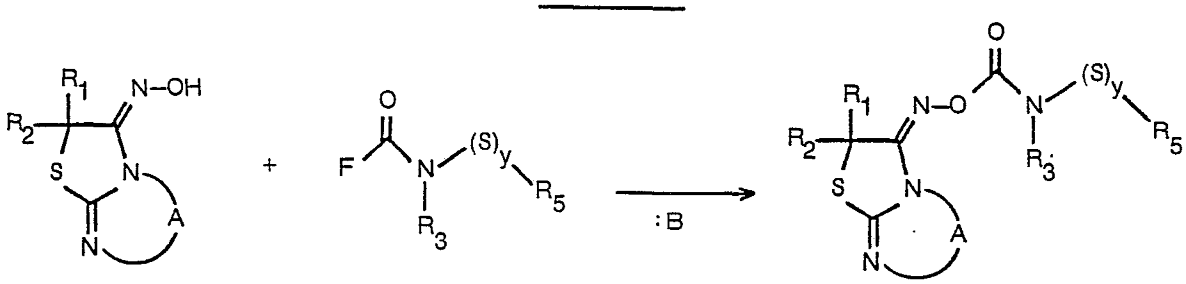


METHOD VI

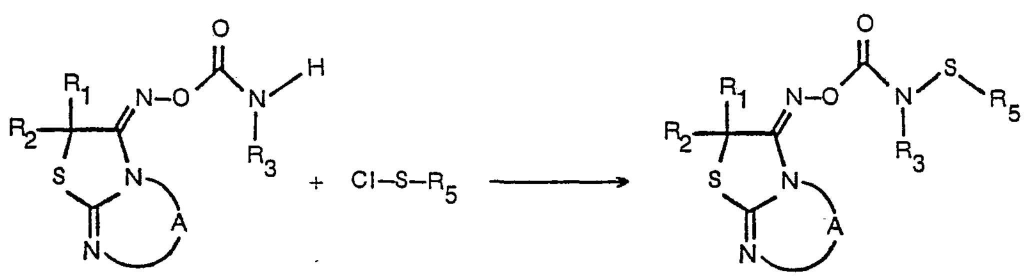

In the above reactions methods, $R_1$, $R_2$ and A are as indicated previously:

n = 0, 1 or 2;

$R_3$ and $R_4$ may be the same or different and are individually hydrogen, $C_1$—$C_4$ alkyl or $C_1$—$C_4$ alkanoyl which can be substituted with one or more halogens and in the case when $R_3$ is alkyl $R_4$ can also be —$(S)_y$—$R_5$ wherein:

Y = 1 or 2;

$R_5$ = a) $C_1$—$C_{18}$ alkyl, $C_3$—$C_8$ cycloalkyl or perhaloalkyl.

b) dialkyl amino, piperidino, pyrrolidino, or a morpholino group each of which may be unsubstituted or substituted with one or two alkyl groups; or

c) a phenyl group which may be unsubstituted or substituted with one or more alkyl, chloro, fluoro, cyano, nitro, alkoxy, trifluoromethyl, alkylthio, alkylsulfinyl, alkylsulfonyl, alkoxy alkyl, alkylthioalkyl, alkylsulfinylalkyl, alkylsulfonylalkyl, or dialkylamino: or

d) a group of the formula

A. 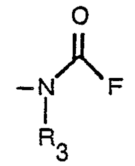 B. 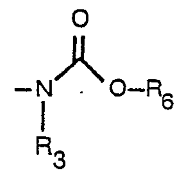

wherein:

$R_6$ is:

1) $C_1$—$C_{12}$ alkyl, alkoxyalkyl, or a phenylalkyl group; or

2) a phenyl group which may be unsubstituted or substituted with one or more $C_1$—$C_{12}$ alkyl, chloro, fluoro, alkoxy, alkylthio, alkylthioalkyl, alkylsulfinyl, alkylsulfinylalkyl, alkylsulfonyl, alkyl sulfonylalkyl, alkynyloxy, dialkylamino, alkoxy imino, formamidino, cyano, dioxolanyl, or dithiolanyl groups in any combination; or

3) a naphthyl, tetrahydronaphthyl, dihydrobenzofuranyl, benzodioxolanyl, or benzothienyl group all of which may be unsubstituted or substituted with one or more alkyl groups; or

4) a group of the formula

i, 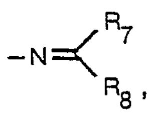 or ii, 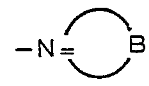

wherein:

$R_7$ is chloro, alkyl, alkoxy, alkylthio, cyanoalkylthio, amidoalkylthio or cyano group; or $R_7$ is hydrogen provided $R_8$ is not hydrogen;

$R_8$ is an alkyl, alkylthio, alkoxycarbonyl, aminocarbonyl, alkylaminocarbonyl, dialkylaminocarbonyl, alkylthioalkyl, cyanoalkyl, alkylsulfinylalkyl, alkyl-sulfonylalkyl, alkoxyalkyl, nitroalkyl, hydroxyalkyl, haloalkyl or phenyl group; said phenyl group may contain one or more alkyl, chloro, or fluoro groups in any combination; or $R_8$ is hydrogen provided $R_7$ is not hydrogen;

B is a divalent aliphatic chain completing a five or six membered heterocyclic ring structure, said aliphatic chain having from 2 to 24 aliphatic carbon atoms, said ring structure may include in any combination one, two or three divalent oxygen, sulfur, sulfinyl, or sulfonyl groups and may further include one group selected from a divalent amino group, a $C_1$—$C_8$ divalent alkylamino group, or a divalent carbonyl group:

with the proviso that when $R_6$ is a phenyl group substituted with an alkyl group, no single alkyl or alkylene moiety in any $R_5$, $R_6$, $R_7$, or $R_8$ group can include more than eight carbon atoms except where indicated.

The reactions illustrated in Methods II to VI are conducted in inert solvents. Any inert solvent such as methylene chloride, chloroform, acetonitrile, benzene, toluene, xylene, dioxane, tetrahydrofuran or a mixture of these solvents can be used in these reactions. Preferably these reactions are conducted in methylene chloride or methylene chloride/acetonitrile, depending on solubility characteristics of the starting oxime.

Reaction temperatures are not critical and can be varied over a wide range depending to a large extent on solubility of the reactants. The reactions are preferably conducted at a temperature of $-10°$ to 50°C. Reaction pressures are not critical. For convenience these reactions are usually conducted at atmospheric or autogeneous pressure.

The reaction illustrated in Method II is preferably conducted in the presence of a quantity of a catalyst sufficient to provide a suitable and reasonable rate. In general, any conventional catalyst of the type commonly employed to promote reactions between isocyanate compounds and compounds that contain an active hydrogen can be used. Preferred catalysts are tertiary amines such as triethylamine, trimethylamine, pyridine and dibutyltin diacetate.

The reaction illustrated in Method III is conducted in the presence of one equivalent of a base such as triethylamine, trimethylamine, pyridine, or potassium hydroxide. When an inorganic base is used phase transfer agents may be used to facilitate the transfer of the base across the organic/inorganic phase interface. Ilustrative of useful phase transfer agents are crown ether compounds, and quaternary ammonium halide compounds. The chloro compound starting reactant is commercially available or easily prepared from known procedures.

The reaction illustrated in Method IV is an oxidation reaction of the product of Method III. The peracetic acid is admixed with the starting reactant and stirred in ethyl acetate at room temperature until the product is produced.

The reaction illustrated in Method V is conducted in the presence of an acid acceptor. The molar ratio of acid acceptor to either reactant is usually unity although a slight excess of acid acceptor may be employed, if desired. The acid acceptor utilized in the conduct of the reaction of Method V may be either an organic or inorganic base. Aromatic and aliphatic tertiary amines, such as triethylamine,

pyridine, and 1,4-diazobicyclo[2.2.2]octane are representative of the organic bases. Base such as sodium hydroxide and potassium carbonate are illustrative of the inorganic bases which are useful in the conduct of this reaction.

When an inorganic base is used phase transfer agents may be used to facilitate the transfer of the base across the organic/inorganic phase interface. Illustrative of useful phase transfer agents are crown ether compounds and quaternary ammonium halide compounds.

The alkylsulfenyl compounds of Method VI are prepared by reacting the products of Method I with an alkylsulfenylchloride in the presence of an organic or inorganic base. Aromatic and aliphatic tertiary amines, such as triethylamine, pyridine, and 1,4-diazobicyclo[2.2.2]octane are representative of the organic bases. Bases such as sodium hydroxide and potassium hydroxide are illustrative of the inorganic bases which are useful in the conduct of this reaction.

When an inorganic base is used phase transfer agents may be used to facilitate the transfer of the base across the organic/inorganic phase interface. Illustrative of useful phase transfer agents are crown ether compounds and quaternary ammonium halide compounds.

The 2-chlorohydroxamoyl chloride compounds of Method I can be prepared by the method described in *Ogloblin et al*, J. Gen. Chem. USSR, 34, 1225 (1964).

The sulfenylated reactant of Method V can be conveniently prepared by reacting hydrogen fluoride with an alkyl isocyanate to form the N-alkylcarbamoyl fluoride compound which is then reacted with an appropriate sulfenyl chloride, e.g. with sulfur dichloride ($SCl_2$) to form the bis-(N-alkyl-N-fluorocarbonylamino)sulfide. This particular material is then reacted with one equivalent of an appropriately substituted alcohol or oxime in the presence of an acid acceptor to form the corresponding mono-fluoro adduct. The carbamoyl fluorides are described in U.S. Patents 4,091,016 and U.S. 3,639,471; Belgium Patents 848,914 and 848,910; and Germany Offen. 2,813,374.

The thioureas used as reactants in Method I are known materials which can be obtained from commercial sources or prepared in accordance with conventional methods known to those skilled in the art.

The following compounds are representative of compounds which come within the contents of the generic formula and which can be prepared according to Method I.

2,2-Dimethyl-3-hydroxyimino-[5,8]-methano-2,3,5,6,7,8-hexahydrothiazolo[3,2-a][1,3]diazepine;
2,2-Dimethyl-3-hydroxyimino-[5,8]-methano-2,3,5,8-tetrahydrothiazolo[3,2-a][1,3]diazepine;
2,2-Dimethyl-3-hydroxyimino-5-oxo-2,3,5,6,7,8-hexahydrothiazolo[3,2-a]benzimidazole;
2,2-Dimethyl-3-hydroxyimino-2,3,4,5,6,7-hexahydrothiazolo[3,2-c][1,3,5]thiadiazine;
2,2-Dimethyl-3-hydroxyimino-2,3-dihydro-9*H*-thiazolo[3,2-a]quinazoline;
5,6-Dichloro-2,2-dimethyl-3-hydroxyimino-2,3-dihydroimidazo[2,1-b]thiazole;
5,6-Dicyano-2,2-dimethyl-3-hydroxyimino-2,3-dihydroimidazo[2,1-b]thiazole;
2,2-Dimethyl-3-hydroxyimino-5-oxo-2,3-dihydro-5*H*-thiazolo[3,2-a]pyrimidine;
5,5-Dimethyl-6-hydroxyimino-2,3-dihydrothiazolo[3,2-d]tetrazole;
2,2-Dimethyl-3-hydroxyimino-2,3,4a,6,7,7a-hexahydro-5*H*-cyclopenta[4,5]imidazo[2,1-b]-thiazole;
2,2-Dimethyl-3-hydroxyimino-2,3-dihydro-6*H*-thiazolo[3,2-b][1,2,4]oxadizole;
7,7-Dichloro-2,2-dimethyl-3-hydroxyimino-2,3-dihydrothiazolo[3,2-a]pyrimidine;
6,6-Dichloro-2,2-dimethyl-3-hydroxyimino-2,3,5,6-tetrahydro-7*H*-thiazolo[3,2-a]pyrimidine;
2,2-Dimethyl-3-hydroxyimino-5,7-dioxo-2,3-dihydro-5*H*-7*H*-thiazolo[3,2-c]oxadiazine;
2,2-Dimethyl-5-methoxy-3-hydroxyimino-2,3-dihydroimidazo[2,1-b]thiazole;
3-Hydroxyimino-2,3,5,6-tetrahydro-7*H*-thiazolo[3,2-a]pyrimidine;
2-Ethyl-3-hydroxyimino-2,3-dihydroimidazo[2,1-b]thiazole;
2-Trichloromethyl-3-hydroxyimino-2,3-dihydroimidazo[2,1-b]thiazole;
2-Isopropyl-3-hydroxyimino-2,3,5,6-tetrahydro-7*H*-thiazolo[3,2-a]pyrimidine; and
2,2-Dimethyl-3-hydroxyimino-2,3-dihydroimidazo[2,1-b]thiazole-1,1-dioxide.

The following insecticidally active compounds are representative of compounds which can be prepared from the compounds of this invention by selecting appropriate starting materials for use in the procedures described above.

2,2-Dimethyl-3-[O-(methylcarbamoyl)oximino]-[5,8]-methano-2,3,5,6,7,8-hexahydrothiazolo[3,2-a]-[1,3]diazepine.
2,2-Dimethyl-3-[O-(methylcarbamoyl)oximino]-[5,8]-methano-2,3,5,8-tetrahydrothiazolo[3,2-a]-[1,3]diazepine.
2,2-Dimethyl-3-[O-(methylcarbamoyl)oximino]-5-oxo-2,3,5,6,7,8-hexahydrothiazolo[3,2-a]-benzimidazole.
2,2-Dimethyl-3-[O-(methylcarbamoyl)oximino]-2,3,4,5,6,7-hexahydrothiazolo[3,2-c]-[1,3,5]thiadiazine.
2,2-Dimethyl-3-[O-(methylcarbamoyl)oximino]-2,3-dihydro -9*H*-thiazolo[3,2-a]quinazoline.
5,6-Dichloro-2,2-dimethyl-3-[O-(methylcarbamoyl)oximino]-2,3-dihydroimidazo[2,1-b]thiazole.
5,6-Dicyano-2,2-dimethyl-3-[O-(methylcarbamoyl)oximino]-2,3-dihydroimidazo[2,1-b]thiazole.
2,2-Dimethyl-3-[O-(methylcarbamoyl)oximino]-5-oxo-2,3-dihydro-5*H*-thiazolo[3,2-a]pyrimidine.
5,5-Dimethyl-6-[O-(methylcarbamoyl)oximino]-2,3-dihydrothiazolo[3,2-d]tetrazole.

2,2-Dimethyl-3-[O-(methylcarbamoyl)oximino]-2,3,4a,6,7,7a-hexahydro-5*H*-cyclopenta[4,5]-imidazo[2,1-b]thiazole.

2,2-Dimethyl-3-[O-(methylcarbamoyl)oximino]-2,3-dihydro-6*H*-thiazolo[3,2-b][1,2,4]oxadiazole.

7,7-Dichloro-2,2-dimethyl-3-[O-(methylcarbamoyl)oximino]-2,3-dihydrothiazolo[3,2-a]-pyrimidine.

6,6-Dichloro-2,2-dimethyl-3-[O-(methylcarbamoyl)oximino]2,3,5,6-tetrahydro-7*H*-thiazolo[3,2-a]-pyrimidine.

2,2-Dimethyl-3-[O-(methylcarbamoyl)oximino]-5,7-dioxo-2,3-dihydro-5*H*,7*H*-thiazolo[3,2-c]-oxadiazine.

2,2-Dimethyl-5-methoxy-3-[O-(methylcarbamoyl)oximino]-2,3-dihydroimidazo[2,1-b]thiazole.

3-[O-(methylcarbamoyl)oximino]2,3,5,6-tetrahydro-7*H*-thiazolo[3,2-a]pyrimidine.

2-Ethyl-3-[O-(methylcarbamoyl)oximino]-2,3-dihydroimidazo[2,1-b]thiazole.

2-Trichloromethyl-3-[O-(methylcarbamoyl)oximino]-2,3-dihydroimidazo[2,1-b]thiazole.

2-Isopropyl-3-[O-(methylcarbamoyl)oximino]-2,3,5,6-tetrahydro-7*H*-thiazolo[3,2-a]pyrimidine.

2,2-Dimethyl-3-[O -(methylcarbamoyl)oximino]-2,3-dihydroimidazo[2,1-b]thiazole-1,1-dioxide.

2,2-Dimethyl-2,3,5,6-tetrahydro-7*H*-3-[[O-[N-methyl-N-(N′-methyl-N′-((2-methyl-2-(methylthio-propylidene)aminooxycarbonyl)aminosulfenyl)carbamoyl]oximino]]thiazolo[3,2-a]pyrimidine.

2,2-Dimethyl-2,3-dihydro-3[[O-[N-methyl-N-(N′-methyl-N′-(2,3-dihydro-2,2-dimethylbenzofuran-7-oxo-carbonyl)aminosulfenyl)carbamoyl]oximino]]imidazo[2,1-b]thiazole.

2,2-Dimethyl-2,3-dihydro-3-[[O-[N-methyl-N-(N′-methyl-N′-((1-N,N-dimethylcarbamoyl-1-methylthiomethylidene-aminooxycarbonyl))aminosulfenyl)carbamoyl]oximino]]imidazo[2,1-b]thiazole.

2,2-Dimethyl-2,3,5,6-tetrahydro-7*H*-3[[O-[N-methyl-N-(N′-methyl-N′-(1-N,N-dimethylcarbamoyl-2-methoxy-iminopropylideneamino oxycarbonyl)aminosulfenyl)carbamoyl]oximino]]thiazole[3,2-a]pyrimidine.

2,2-Dimethyl-2,3-dihydro-3[[O-[N-methyl-N-(morpholinosulfenyl)carbamoyl]oximino]]imi-dazo[2,1-b]thiazole.

2,2-Dimethyl-2,3,5,6-tetrahydro-7*H*-3[[O-[N-methyl-N-(4-*tert*-butylphenyl sulfenyl)carbamoyl]oximino]]thiazolo[3,2-a]pyrimidine.

2,2-Dimethyl-2,3-dihydro-3-[[O-[N-methyl-N-(4-methylthio phenyl sulfenyl)carbamoyl]ox-imino]]imidazo[2,1-b]thiazole.

2,2-Dimethyl-2,3,5,6-tetrahydro-7*H*-3[[O-[N-methyl-N-(N′-methyl-N′-((*tert*-butoxycarbonyl]amino sulfenyl)carbamoyl]oximino]]thiazolo[3,2-a]pyrimidine.

2,2-Dimethyl-2,3-dihydro-3-[[O-[N-methyl-N-(*tert*-butylthiosulfenyl)carbamoyl]oximino]]imi-dazo[2,1-b]thiazole.

2,2-Dimethyl-2,3,5,6-tetrahydro-7*H*-3-[[O-[N-methyl-N-(4-*tert*-butylphenylthiosulfenyl)carbamoyl]oximino]]thiazolo[3,2-a]pyrimidine.

2,2-Dimethyl-3-[O-carbamoyloximino]2,3-dihydroimidazo[2,1-b]thiazole.

3-[2,2-Dimethyl-2,3-dihydroimidazo[2,1-b]thiazol-3-ylidenamino-oxycarbonyl methylaminothio-sulfenylmethyl carbamoyloxyimino]-2,2-dimethyl-2,3-dihydroimidazo[2,1-b]thiazole.

All compounds with the the purview of the above generic formula can be used as precursors for the preparation of insecticidal compounds which exhibit insecticidal activity to a lesser or greater extent. Some of the insecticidal compounds exhibit very powerful insecticidal activity in extremely small dosages while others require larger dosages to be insecticidally effective.

Preferred because of their use in preparing insecticidal compounds which exhibit high levels of insecticidal activity are those of the above generic structural formula wherein:

$R_1$ and $R_2$ are individually $C_1$—$C_6$ alkyl and most preferably methyl;

n is 0 or 1 and

A is an acyclic alkylene or alkenylene group, or an ortho-substituted phenylene group which forms an unsaturated five, six or seven membered heterocyclic ring which also may contain nitrogen or carbonyl in addition to the two nitrogens, said alkylene, alkenylene, or phenylene ring systems being optionally substituted with one or more $C_1$—$C_4$ alkyl or cyclohexyl. Most preferably A would constitute part of the following ring systems, pyrimidine, diazepine, triazole, imidazole, and benzimidazole.

The following specific examples are presented to more particularly illustrate this invention. Examples I—IX show the preparation of the compounds of the instant invention whereas Examples X to XXVII show insecticidal compounds produced from the compounds of the instant invention.

Example I

Preparation of 2,2-Dimethyl-3-hydroxyimino-2,3,5,6-tetrahydroimidazo[2,1-b]thiazole

A 10.2 g (65.4 mmol) of 2-chlorohydroxyamoyl chloride was dissolved in 200 ml of methanol and immediately cooled in an ice bath to 0°. To this solution was added 6.68g (65.4 mmol) of 2-imidazolidinethione in one portion. After the reaction mixture was stirred at 0° for ca. 10 min., 11.09 g (132 mmol) of sodium bicarbonate was added in portions. The reaction mixture was allowed to stir at 25°C overnight before the solvent was removed *in vacuo.* The residue was extracted with absolute ethanol and the ethanolic extracts were concentrated *in vacuo*. The residue was triturated with cold acetonitrilehexane. The insoluble solid (22%) was identified as the desired product:

mp: 188—191°. nmr (5% DMSO-d6) δ 9.75 (br,1,NO*H*), 4.10 (brs, 4, —N—$CH_2CH_2$N—), 3.50—3.30 (m,4,$2H_2O$), 1.67 (s, 6, gem-dimethyl). ir ($CH_2Cl_2$) 3570, 1670, 1620, 1200, 920 $cm^{-1}$.

The following Examples II—IX were prepared in a manner similar to Example I. The nomenclature and structure of the compounds of each example are as follows:

| Example | Nomenclature | Structure |
|---|---|---|
| II | 2,2-Dimethyl-3-hydroxyimino-2,3,5,6-tetrahydro-7*4*-thiazolo[3,2,-a]pyrimidine | 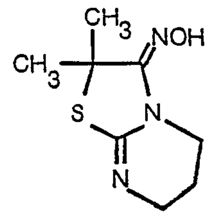 |
| III | 2,2,5,5,8,8-Hexamethyl-3-hydroxyimino 2,3,5,6,7,8-hexahydrothiazolo [3,2-a][1,3]diazepine | 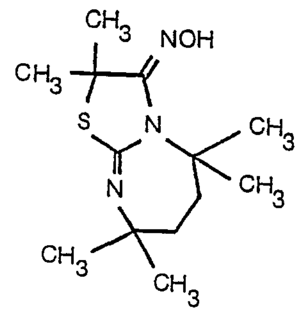 |
| IV | 6,6-Dimethyl-5-hydroxyimino-5,6-dihydrothiazolo[2,3-c]1,2,4-triazole | 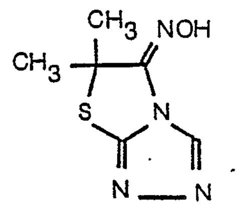 |
| V | 2,2-Dimethyl-3-hydroxyimino-2,3-dihydroimidazo[2,1-b]thiazole | 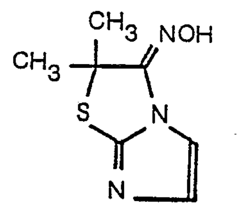 |
| VI | 6,6-Dimethyl-3-hydroxyimino-2,3-dihydrothiazolo[3,2-a]benzimidazole | 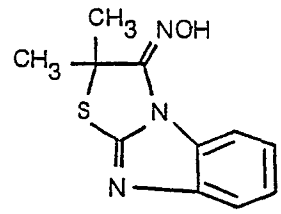 |
| VII | *E*-2,2-Dimethyl-3-hydroxyimino-7-oxo-2,3-dihydro-7*H*-thiazolo [3,2-a]pyrimidine | 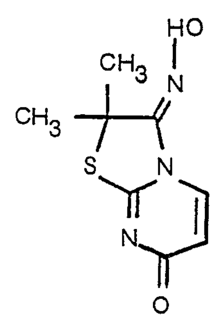 |

| Example | Nomenclature | Structure |
|---|---|---|
| VIII | 2,2-Dimethyl-3-hydroxyimino-5-oxo-2,3-dihydro-5*H*-thiazolo [3,2-a]pyrimidine | 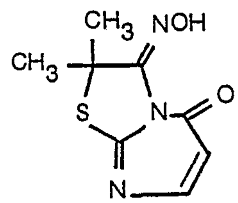 |
| IX | *Z*-2,2-Dimethyl-3-hydroxyimino-7-oxo-2,3-dihydro-7*H*-thiazolo[3,2-a] pyrimidine | 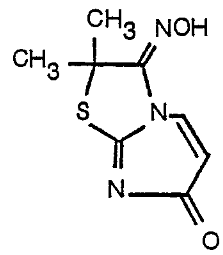 |

The physical data for these compounds are as indicated in Table I.

TABLE I

| Example | MP—°C | nmr/Anal calcd | Found |
|---|---|---|---|
| II | 191—193° | C—48.22<br>H— 6.57<br>N—21.09 | 47.72<br>6.30<br>21.04 |
| III | 161—163° | nmr (5% DMSO-$d_6$) $\delta$<br>1.90—1.50 (m,10,singlet at 1.60 $\delta$ for *gem*-dimethyl)<br>1.35 (s, 6, N 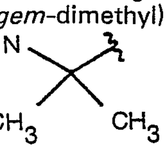<br>1.18 (s, 6, =N 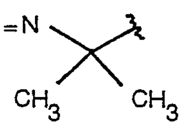 | |
| IV | 202—204° | nmr (DMSO-$d_6$) $\delta$<br>12.00 (s, I, NOH)<br>9.20 (s, I, C—H)<br>1.82 (s, 6, gem-dimethyl) | |
| V | 202.5—204° | C—45.88<br>H— 4.95<br>N—22.93 | C—46.17<br>H— 4.83<br>N—23.08 |
| VI | — | nmr (DMSO-$d_6$) $\delta$<br>11.72 (s, I, NOH)<br>8.30—7.10 (m, 4, aromatic)<br>1.82 (s, 6, gem dimethyl) | |

TABLE 1 (continued)

| Example | MP—°C | nmr/Anal calcd | Found |
|---|---|---|---|
| VII | >240° | C—45.48<br>H— 4.29<br>N—19.88 | C—45.56<br>H— 4.20<br>N—20.23 |
| VIII | 132—143° | C—45.48<br>H— 4.29<br>N—19.89 | C—45.47<br>H— 4.26<br>N—19.99 |
| IX | — | nmr (DMSO-$d_6$) $\delta$<br>12.12 (s, l, OH)<br>8.90 (d, l, CO CH=*CH*, *J*=8 Hz)<br>6.00 (d, l, CO CH=CH, *J*=8 Hz)<br>1.78 (s, 6, gem-dimethyl) | |

Example X

Preparation of 2,2-Dimethyl-3-[0(methylcarbamoyl)oximino]-2,3,5,6-tetrahydroimidazo[2,1-b]thiazole

To a solution of 2.63 g (14.2 mmol) of 2,2-dimethyl-3-hydroxyimino-2,3,5,6-tetrahydroimidazo[2,1-b] thiazole (I) in 200 ml of a 1:1 mixture of methylene chloride:acetonitrile, taken in a pyrex pressure bottle, was added 1.13 ml (15 mmol) of methylisocyanate and two drops of di-butyltindiacetate. After standing at 25° for 24 hours the solvent was removed *in vacuo* and the resulting oil was crystallized from hexane/toluene to afford 1.5 g (44%) of the product. mp 142—147°.

Calc'd for $C_9H_{14}N_4O_2S$: C, 44.61; 4, 5.82; N, 23.12.

Found: C, 44.24; 4, 5.81; N, 23.11

The following examples XI—XVII were prepared in a manner similar to Example X. The nomenclature and structure of the compounds of each example are as follows:

| Example | Nomenclature | Structure |
|---|---|---|
| XI | 2,2-Dimethyl-3-[0-(methylcarbamoyl)-oximino]-2,3,5,6-tetrahydro-7*H*-thiazolo [3,2-a]pyrimidine | 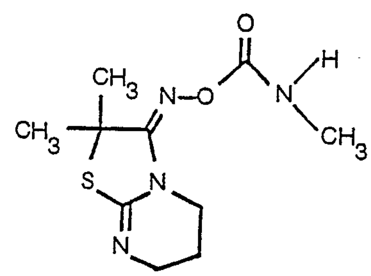 |
| XII | 2,2,5,5,8,8-Hexamethyl-3-[0-(methyl-carbamoyl)oximino]-2,3,5,6,7,8-hexahydrothiazolo[3,2-a][1,3]diazepine | 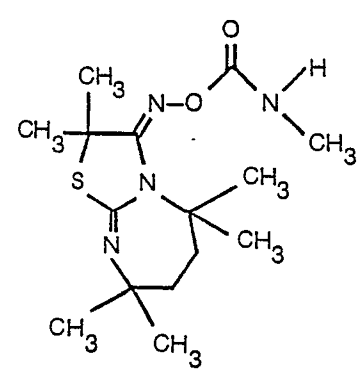 |

| Example | Nomenclature | Structure |
| --- | --- | --- |
| XIII | 6,6-Dimethyl-5-[0-(methylcarbomoyl)-oximino]-5,6-dihydrothiazolo[2,3-c] 1,2,4-triazole | 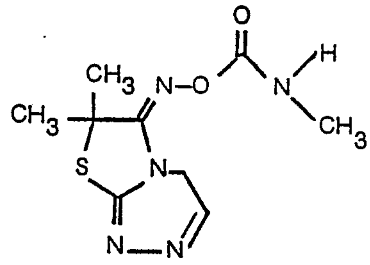 |
| XIV | 2,2-Dimethyl-3-[0-(methylcarbamoyl)-oximino]-2,3-dihydroimidazo[2,1-b] thiazole | 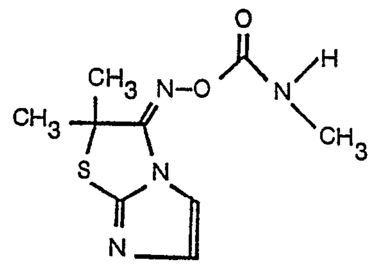 |
| XV | 2,2-Dimethyl-3-[0-(methylcarbamoyl)-oximino]-2,3-dihydrothiazolo[3,2-a] benzimidazole | 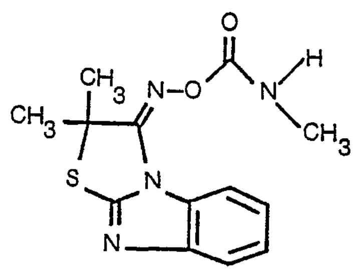 |
| XVI | 2,2-Dimethyl-3-[0-(methylcarbamoyl)-oximino]-7-oxo-2,3-dihydro-7*H*-thiazolo[3,2-a]pyrimidine | 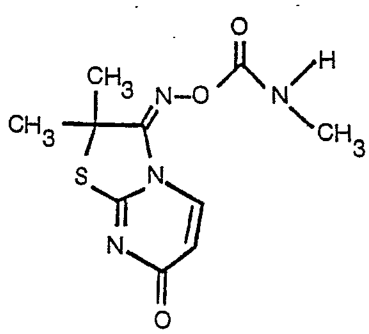 |
| XVII | 2,2-Dimethyl-6*N*-cyclohexyl-3-[0-(methylcarbamoyl)oximino]2,3,5,6-tetrahydro-7*H*-thiazolo[3,2-a]triazine | 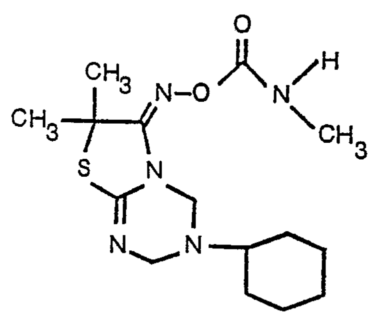 |

The physical data for these compounds are as indicated in Table II.

TABLE II

| Example | MP—°C | nmr/Anal calcd | Found |
|---|---|---|---|
| XI | 142.5—144° | C—46.85<br>H— 6.29<br>H—21.86 | C—46.96<br>H— 6.39<br>N—22.19 |
| XII | 141—143° | C—55.18<br>H— 8.03<br>N—17.16 | C—55.04<br>H— 8.05<br>N—17.53 |
| XIII | 167—171<br>(dec) | C—39.82<br>H— 4.60<br>N—29.03 | C—39.71<br>H— 4.76<br>N—26.07 |
| XIV | 133—141° | C—44.98<br>H— 5.04<br>N—23.32 | C—44.41<br>H— 5.10<br>N—22.10 |
| XV | 192—195° | C—53.77<br>H— 4.86<br>N—19.30 | C—53.25<br>H— 4.77<br>N—19.05 |
| XVI | 146—149°<br>(dec) | C—44.76<br>H— 4.51 | C—43.70<br>H— 5.54 |
| XVII | 158—161° | C—53.07<br>H— 7.42<br>N—20.63 | C—51.03<br>H— 7.18<br>N—20.76 |

The following examples XVIII to XXII further illustrate preparation of additional insecticidal compounds with Examples XIX and XXII being prepared in a manner similar to Example XVIII by adding one equivalent of appropriately substituted alcohol or oxime to the bis-(N-alkyl-N-fluorocarbonyl-amino) sulfide in the presence of an acid acceptor followed by an equivalent of oxime.

The nomenclature and structure of the compounds of each example are as indicated below.

Example XVIII

Preparation of 3-[2,2-Dimethyl-2,3-dihydroimidazo[2,1-b]thiazol-3-ylidenamino-oxycarbonyl methyl-aminosulfenylmethyl carbamoyloxyimino]-2,2-dimethyl 2,3-dihydroimidazo[2,1-b]thiazole

A 3.0 g (16.4 mmol) quantity of 2,2-dimethyl-3-hydroxyimino-2,3,5,6-tetrahydroimidazo[2,1-b]thiazole (I) was dissolved in 300 ml of methylene chloride containing 2.28 ml (16.4 mmol) of triethylamine. To this mixture was added 1.5 g (8.2 mmol) of bis-(N-alkyl-N-fluorocarbonylamino) sulfide and the reaction mixture was stirred overnight. The reaction mixture was washed with water (2 × 200 ml), dried ($MgSO_4$), and concentrated in vacuo to give a solid residue which was recrystallized from ethylacetate. The final product crystallized with one equivalent of solvent (ethylacetate or acetone) per two equivalents of compound. Yield 3.24g (65%); mp. 177—180°

Calcd for $[C_{18}H_{22}N_8O_4S_3]_2$ $C_4H_8O_2$: C, 43.30, H, 4.73; N, 20.20

Found: C, 42.82, H, 4.70; N, 20.1

| Example | Nomenclature | Structure |
|---|---|---|
| XIX | 2,2-Dimethyl-2,3-dihydro-3-[[0-[N-methyl-N-(N′-methyl-N′-(1-methylthio-ethylidene aminooxycarbonyl) aminosulfenyl) carba-moyl]oximino]] imidazo [2,1-b]thiazole | 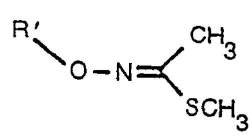 |

| Example | Nomenclature | Structure |
|---|---|---|
| XX | 2,2-Dimethyl-2,3-dihydro-3-[[O-[N-methyl-N-(N′-methyl-N′-(naphthaleneoxy carbonyl) amino sulfenyl)carbamoyl]oximino]]imidazo[2,1-b] thiazole | 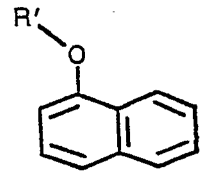 |

R′ = 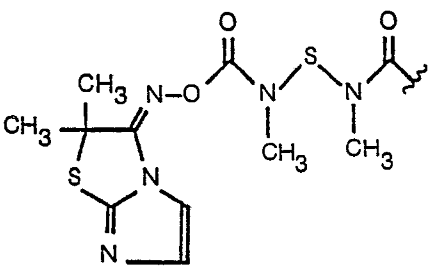

| Example | Nomenclature | Structure |
|---|---|---|
| XXI | 2,2-Dimethyl-2,3-dihydro-3-[[O-[N-methyl-N-(N′-methyl-N′-(3,4,5-trimethylphenoxycarbonyl) amino sulfenyl)carbamoyl]oximino]]imidazo [2,1-b]thiazole | 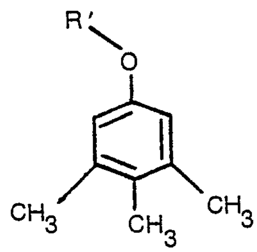 |
| XXII | 2,2-Dimethyl-2,3-dihydro-3[O-[N-methyl-N-(N′-methyl-N′-(4-*n*-nonelphenoxycarbonyl) aminosulfenyl)carbamoyl]oximino]imidazo [2,1-b]thiazole | 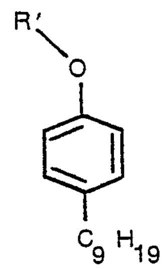 |

TABLE III

| Example | MP | Analysis Calc | Analysis Found |
|---|---|---|---|
| XIX | 50—55° | C—38.87<br>H— 4.66<br>N—19.43 | C—38.19<br>H— 4.83<br>N—18.56 |
| XX | 85° (dec) | C—53.48<br>H— 4.49<br>N—14.85 | C—52.91<br>H— 4.65<br>N—14.00 |
| XXI | 131.5—134° | C—51.81<br>H— 5.44<br>N—15.11 | C—51.61<br>H— 5.41<br>N—15.09 |
| XXII | gummy solid | C—57.01<br>H— 6.81<br>N—12.79 | C—56.64<br>H— 6.86<br>N—12.60 |

Example XXIII

Preparation of 2,2-Dimethyl-2,3-dihydro-3[0-(dimethyl carbamoyl)oximino]-imidazo-[2,1-b]thiazole

To a solution of 330 mg (13.6 mmol) of sodium hydride in 150 ml of dry tetrahydrofuran was added 2.50 g (13.6 mmol) of 2,2-dimethyl-3-hydroxyimino-2,3-dihydroimidazo-[2,1-b]thiazole. The reaction mixture was stirred at 25° for 1 hour and cooled in an ice bath before 1.54 g (14.3 mmol) of dimethylcarbamoyl chloride was added. After stirring for 12 hours at 25°, the solvent was removed in vacuo and the residue washed with hexane, dissolved in methylene chloride. The methylene chloride solution was washed with water, dried ($MgSO_4$), and concentrated in vacuo to give 2.94 g (85%) of desired product. m.p. 99—102.5°.

Anal calc'd for $C_{10}H_{14}N_4O_2S$: C, 47.23; H, 5.55; N, 22.03

Found: C, 47.65; H, 5.71; N, 21.89

The following examples XXIV—XXVI further illustrate preparation of additional insecticidal compounds. Example XXIV was prepared according to the procedure of Example XXIII. Example XV was prepared according to the procedure of Example X and Example XXVI was prepared according to the procedure of Example XVIII.

The nomenclature and structure of each example are as indicated below.

| Example | Nomenclature | Structure |
|---|---|---|
| XXIV | 2,2-Dimethyl-2,3-dihydro-3-[0-(N-methyl-N-pentanoyl carbamoyl)oximino]-imidazo [2,1-b]thiazole | 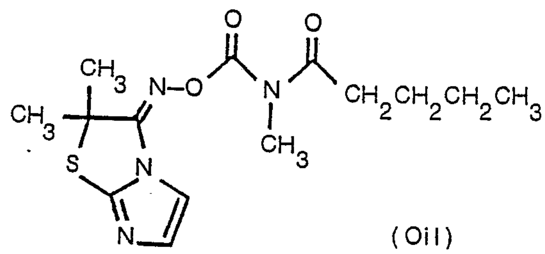 (Oil) |
| XXV | 2,2-Dimethyl-2,3-dihydro-3-[0-(N-propyl carbamoyl)oximino]-imidazo[2,1-b]thiazole | 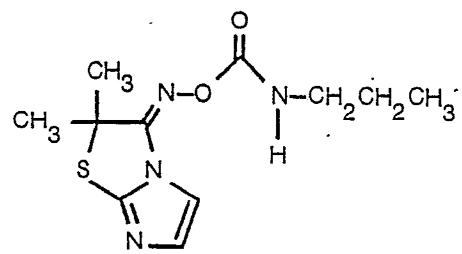 |
| XXVI | 2,2-Dimethyl-2,3-dihydro-3[0-(N-methyl-N- (4-*tert*-butylphenyl-thiosulfenyl)carbamoyl)oximino]-imidazo[2,1-b]thiazole | 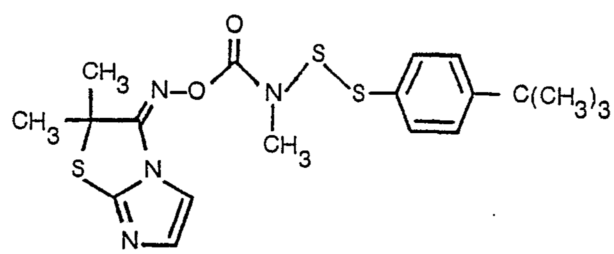 |

The physical data for Examples XXIV—XXVI are as indicated in Table IV.

TABLE IV

| Example | MP—°C | Elemental calcd | Anal. Found |
|---|---|---|---|
| XXIV | | C—51.83<br>H— 6.21<br>N—17.27 | C—51.87<br>H— 6.23<br>N—16.88 |
| XXV | 103.5—105° | C—49.23<br>H— 6.01<br>N—20.88 | C—49.26<br>H— 5.96<br>N—21.13 |
| XXVI | | C—52.26<br>H— 5.54<br>N—12.83 | C—51.53<br>H— 5.50<br>N—12.66 |

Example XXVII

Preparation of 2,2-Dimethyl-3-[0-(methylcarbamoyl) oximino]-2,3-dihydroimidazo [2,1-b] thiazole-1-oxide

A 2.0 g (8.32 mmol) quantity of 2,2-dimethyl-3-[0-(methyl carbamoyl) oximino]-2,3-dihydroimidazo [2,1-b] thiazole was dissolved in 300 ml of ethylacetate. The solution was cooled to —15° before 1.33 g (17.5 mmol) of peracetic acid was added. The reaction mixture was allowed to warm to 25° and stirred for 1 d, followed by concentration to ca. 100 ml and placing in the freezer overnight. The precipitate was collected to give 1.5 g (70%) of product. m.p. 147.5—149°.

Anal. calc'd for $C_9H_{12}N_4O_3S$: C, 42.18; H, 4.72; N, 21.86.
Found: C, 41.91; H, 4.75; N, 21.65.

Selected new compounds were evaluated to determine their pesticidal activity against mites, nematodes, and certain insects, including an aphid, a caterpillar, a beetle and a fly.

Suspensions of the test compounds were prepared by dissolving one gram of compound in 50 milliliters of acetone in which had been dissolved 0.1 gram (10 percent of the weight of compound) of an alkylaryl polyether alcohol surfactant, as an emulsifying or dispersing agent. The resulting solution was mixed into 150 milliliters of water to give roughly 200 milliliters of a suspension containing the compound in finely divided form. The thus-prepared stock suspension contained 0.5 percent by weight of compound. The concentrations in parts per million by weight employed in the tests described below were obtained by appropriate dilutions of the stock suspension with water. The test procedures were as follows:

Adults and nymphal stages of the bean aphid (*Aphis fabae* Scop.) reared on potted dwarf nasturtium plants at 18.3—21.1°C (65—70°F) and 50 ± 5 percent relative humidity, constituted with test insects. For testing purposes, the number of aphids per pot was standardized to 100—150 by trimming plants containing excess aphids.

The test compounds were formulated by diluting the stock suspension with water to give a suspension containing 500 parts of test compound per million parts of final formulation.

The potted plants (one pot per compound tested) infested with 100—150 aphids, were placed on a revolving turntable and sprayed with 100—110 milliliters of test compound formulated by use of a DeVilbiss spray gun set at 276 kn/m² (40 psig) air pressure. This application, which lasted 25 seconds, was sufficient to wet the plants to run-off. As a control, 100—110 milliliters of a water-acetone-emulsifier solution containing no test compound were also sprayed on infested plants. After spraying, the pots were placed on their sides on a sheet of white standard mimeograph paper which had been previously ruled to facilitate counting. Temperature and humidity in the test room during the 24 hour holding period were 18.3—21.1°C (65—70°F) and 50—70 percent, respectively. Aphids which fell onto the paper and were unable to remain standing after being uprighted were considered dead. Aphids remaining on the plants were observed closely for movement and those which were unable to move the length of the body upon stimulation by prodding were considered dead.

Larvae of the southern armyworm (*Spodopteraeridania,* (Cram.)), reared on Tendergreen bean plants at a temperature of 26.7 ± 2.8°C (80 ± 5°F) and a relative humidity of 50 ± 5 percent, constituted the test insects.

The test compounds were formulated by diluting the stock suspension with water to give a suspension containing 500 parts of test compound per million parts of final formulation. Potted Tendergreen bean plants of standard height and age were placed on a revolving turntable and sprayed with 100—110 milliliters of test compound formulation by use of a DeVilbiss spray gun set at 69 kN/m²

(10 psig) air pressure. This application, which lasted 25 seconds, was sufficient to wet plants to run-off. As a control, 100—110 milliliters of a wateracetone-emulsifier solution containing no test compound were also sprayed on infested plants. When dry, the paired leaves were separated and each one was placed in a 9 centimeter Petri dish lined with moistened filter paper. Five randomly selected larvae were introduced into each dish and the dishes were closed. The closed dishes were labeled and held at 26.7—29.4°C (80—85°F) for three days. Although the larvae could easily consume the whole leaf within twenty-four hours, no more food was added. Larvae which were unable to move the length of the body, even upon stimulation by prodding, were considered dead.

Fourth instar larvae of the Mexican bean beetle (*Epilachna varivestis,* Muls.), reared on Tendergreen bean plants at a temperature of 26.7 ± 2.8°C (80 ± 5°F) and 50 ± 5 percent relative humidity, were the test insects.

The test compounds were formulated by diluting the stock suspension with water to give a suspension containing 500 parts of test compound per million parts of final formulation. Potted Tendergreen bean plants of standard height and age were placed on a revolving turntable and sprayed with 100—110 milliliters of test compound formulation by use of a DeVilbiss spray gun set at 69 kN/m$^2$ (10 psig) air pressure. This application, which lasted 25 seconds, was sufficient to wet plants to run-off. As a control, 100—110 milliliters of a water-acetone-emulsifier solution containing no test compound were also sprayed on infested plants. When dry, the paired leaves were separated and each was placed in a 9 centimeter Petri dish lined with moistened filter paper. Five randomly selected larvae were introduced into each dish, and the dishes were closed. The closed dishes were labeled and held at a temperature of 26.7 ± 2.8°C (80 ± 5°F) for three days. Although the larvae could easily consume the leaf within 24 to 48 hours, no more food was added. Larvae which were unable to move the length of the body, even upon stimulation, were considered dead.

Four to six day old adult house flies (*Musca domestica*, L.), reared according to the specifications of the Chemical Specialties Manufacturing Association (Blue Book, McNair-Dorland Co., N. Y. 1954; pages 243—244, 261) under controlled conditions of 26.7 ± 2.8°C (80 ± 5°F) and 50 ± 5 percent relative humidity, were the test insects. The flies were immobilized by anesthetizing with carbon dioxide and twenty five immobilized individuals, males and females, were transferred to a cage consisting of a standard food strainer about 127 mm (five inches) in diameter which was inverted over a wrapping-paper-covered surface. The test compounds were formulated by diluting the stock suspension with a 10 percent (by weight) sugar solution to give a suspension containing 500 parts of test compound per million parts of final formulation, by weight. Ten milliliters of the test formulation were added to a soufflé cup containing a 25.4 mm (one-inch) square of an absorbent cotton pad. This bait cup was introduced and centered on the blotting paper under the food strainer prior to admitting the anesthetized flies. The caged flies were allowed to feed on the bait for twenty four hours, at a temperature of 26.7 ± 2.8°C (80 ± 5°F) and the relative humidity of 50 ± 5 percent. Flies which showed no sign of movement on prodding were considered dead.

Adults and nymphal stages of the two-spotted mite (*Tetranychus urticae* (Koch)), reared on Tendergreen bean plants at 80 ± 5 percent relative humidity, were the test organisms. Infested leaves from a stock culture were placed on the primary leaves of two bean plants 152 to 203 mm (six to eight inches) in height, growing in a 63.5 mm (two-and-a-half inch) clay pot. 150—200 Mites, a sufficient number for testing, transferred from the excised leaves to the fresh plants in a period of twenty-four hours. Following the twenty-four hour transfer period, the excised leaves were removed from the infested plants. The test compounds were formulated by diluting the stock suspension with water to give a suspension containing 500 parts of test compound per million parts of final formulation. The potted plants (one pot per compound) were placed on a revolving turntable and sprayed with 100—110 milliliters of test compound formulation by use of a DeVilbiss spray gun set at 276 kN/m$^2$ (40 psig) air pressure. This application, which lasted 25 seconds, was sufficient to wet the plants to run-off. As a control, 100—110 milliliters of water solution containing acetone and emulsifier in the same concentration as the test compound formulation, but containing no test compound, were also sprayed on infested plants. The sprayed plants were held at 80 ± 5 percent relative humidity for six days, after which a mortality count of motile forms was made. Microscopic examination for motile forms was made on the leaves of the test plants. Any individual which was capable of locomotion upon prodding was considered living.

The results of these tests are set forth in Table V below. In these tests the pesticidal activity of the compounds at the indicated dosage rate against aphid, mite, Southern Armyworm, Bean Beetle and house fly was rated as follows:

A = excellent control

B = partial control

C = no control

TABLE V

Biological Data

| Example | Bean Aphid | Mite Adult | Southern Armyworm Larvae | Southern Armyworm Egg | Mexican Bean Beetle | House Fly |
|---|---|---|---|---|---|---|
| I | C | C | C | C | C | C |
| VII | C | C | C | C | C | C |
| X | A | A | C | A | A | A |
| XI | A | A | A | A | A | A |
| XII | A | C | C | C | C | B |
| XIII | A | B | B | C | A | A |
| XIV | A | A | A | A | A | A |
| XV | B | C | C | C | C | C |
| XVI | C | C | B | C | C | C |
| XVII | C | C | B | C | C | A |
| XVIII | A | A | A | A | A | A |
| XIX | A | A | A | A | A | A |
| XX | A | A | A | A | A | A |
| XXI | A | A | A | A | A | A |
| XXII | B | C | A | C | A | A |
| XXIII | A | A | A | C | A | A |
| XXIV | A | A | A | C | A | A |
| XXV | A | A | A | B | A | A |
| XXVI | A | A | A | C | A | A |
| XXVII | A | A | A | C | A | A |

It will be understood that the plant species employed in the above tests are merely representative of a wide variety of plant pest that can be controlled by the use of the compounds of this invention. The compounds contemplated in this invention may be applied as mites ovicides and miticides according to methods known to those skilled in the art. Pesticidal compositions containing the compounds as the active toxicant will usually comprise a carrier and/or diluent, either liquid or solid.

Suitable liquid diluents or carriers include water, petroleum distillates, or other liquid carriers with or without surface active agents. Liquid concentrates may be prepared by dissolving one of these compounds with a nonphytotoxic solvent such as acetone, xylene, or nitrobenzene and dispersing the toxicants in water with the aid of suitable surface active emulsifying and dispersing agents.

The choice of dispersing and emulsifying agents and the amount employed is dictated by the nature of the composition and the ability of the agent to facilitate the dispersion of the toxicant. Generally, it is desirable to use as little of the agent as is possible, consistent with the desired dispersion of the toxicant in the spray so that rain does not re-emulsify the toxicant after it is applied to the plant and wash it off the plant. Nonionic, anionic, amphoteric or cationic dispersing and emulsifying agents may be employed; for example, the condensation products of alkylene oxides with phenol and organic

acids, alkyl aryl sulfonates, complex ether alcohols, and quaternary ammonium compounds.

In the preparation of wettable powder or dust or granulated compositions, the active ingredient is dispersed in and on an appropriately divided solid carrier such as clay, talc, bentonite, diatomaceous earth, and fullers earth. In the formulation of the wettable powders the aforementioned dispersing agents as well as lignosulfonates can be included.

The required amount of the toxicants contemplated herein may be applied 0.94 nm/$m^2$—0.188L/$m^2$ (1 to 200 gallons per acre) or more of liquid carrier and/or diluent or in from 0.56 to 56 g/$m^2$ (5 to 500 pounds per acre) of inert solid carrier and/or diluent. The concentration in the liquid concentrate will usually vary from about 10 to 95 percent by weight and in the solid formulations from about 0.5 to about 90 percent by weight. Satisfactory sprays, dusts, or granules for general use contain from 0.028 to 1.68 g/$m^2$ (1/4 to 15 pounds per acre) of active toxicant.

The pesticides contemplated herein prevent attack by insects and mites upon plants or other material to which the pesticides are applied, and they have relatively high residual toxicity. With respect to plants, they have a high margin of safety in that when used in sufficient amount to kill or repel the insects, they do not burn or injure the plant, and they resist weathering which includes wash-off caused by rain, decomposition by ultraviolet light, oxidation, or hydrolysis in the presence of moisture or, at least such decomposition, oxidation, and hydrolysis as would materially decrease the desirable pesticidal characteristic of the toxicants or impart undesirable characteristic for instance, phytotoxicity, to the toxicants. The toxicants are so chemically inert that they are compatible with substantially any other constituents of the spray schedule, and they may be used in the soil, upon the seeds, or the roots of plants without injuring either the seeds or roots of plants. They may also be used in combination with other pesticidally active compounds. When used as miticides they will normally be applied to the foliage of the plants to be treated. It will be appreciated that the compounds of this invention can also be used in combination with biologically active compounds.

## Claims

1. A bicyclic 2-iminothiazolidine oxime of the general formula:

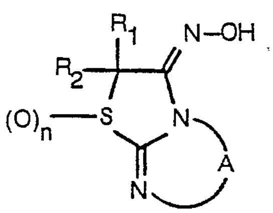

wherein $R_1$ and $R_2$ are the same or different and are individually hydrogen, $C_1$—$C_6$ alkyl or halogen;

n = 0, 1 or 2; and

A is an acyclic, cyclic or bicyclic alkylene or alkenylene group, or an ortho-substituted phenylene group which forms an unsaturated five, six or seven membered heterocyclic ring which can also contain oxygen, sulfur, nitrogen, or carbonyl in addition to the two nitrogens, said alkylene, alkenylene, or phenylene ring systems being optionally substituted with one or more $C_1$—$C_4$ alkyl, cyclohexyl, alkoxy, phenoxy, alkylthio, halogen, amido, alkylamido, nitrile, acyl, or nitro groups.

2. An oxime as claimed in claim 1 wherein $R_1$ and $R_2$ can be the same or different and are individually $C_1$—$C_6$ alkyl.

3. An oxime as claimed in claim 2 wherein $R_1$ and $R_2$ are methyl.

4. An oxime as claimed in any one of claims 1 to 3 wherein n is O.

5. An oxime as claimed in any one of the preceding claims wherein A is an acyclic, cyclic, or bicyclic alkylene or alkenylene group, or an ortho-substituted phenyl group which forms an unsaturated five, six or seven membered heterocyclic ring which also may contain nitrogen or carbonyl in addition to the two nitrogens, said alkylene, alkenylene, or phenylene ring systems being optionally substituted with one or more $C_1$—$C_4$ alkyl or cyclohexyl.

6. An oxime as claimed in claim 5 wherein A forms a pyrimidine, diazepine, triazole, imidazole, or benzimidazole ring system.

7. 2,2-Dimethyl-3-hydroxyimino-2,3,4,6-tetrahydro-imidazo[2,1-b]thiazole.

8. 2,2-Dimethyl-3-hydroxyimino-2,3,4,6-tetrahydro -7*H*-thiazolo[3,2-a]pyrimidine.

9. 2,2,5,5,8,8-Hexamethyl-3-hydroxyimino-2,3,5,6,7,8-hexahydrothiazolo[3,2-a][1,3]diazepine.

10. 6,6-Dimethyl- -5-hydroxyimino-5,6-dihydrothiazolo[2,3-c]1,2,4-triazole.

11. 2,2-Dimethyl-3-hydroxyimino-2,3-dihydroimidazo[2,1-b]thiazole.

12. A method of producing a bicyclic 2-iminothiazolidine oxime as claimed in claim 1, which method comprises reacting a compound of the formula

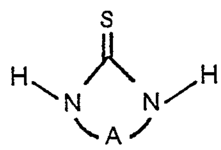

with a compound of the formula

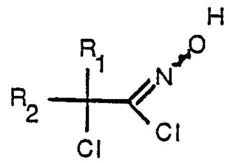

in the presence of an acid acceptor wherein $R_1$, $R_2$, n and A are as defined in claim 1.

**Revendications**

1. Oxime de 2-iminothiazolidine bicyclique de formule générale:

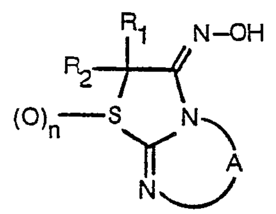

dans laquelle $R_1$ et $R_2$ sont égaux ou différents et représentent individuellement l'hydrogène, un groupe alkyle en $C_1$ à $C_6$ ou un halogène;

*n* est égal à 0, 1 ou 2; et

A est un groupe alkylène ou alcénylène acylique, cyclique ou bicyclique ou un groupe phénylène substitué en ortho qui forme un noyau hétérocyclique pentagonal, hexagonal ou heptagonal insaturé qui peut aussi contenir de l'oxygène, du soufre, de l'azote ou un groupe carbonyle en plus des deux atomes d'azote, lesdits systèmes alkylène, alcénylène ou phénylène cycliques étant éventuellement substitués avec un ou plusieurs groupes alkyle en $C_1$ à $C_4$, cyclohexyle, alkoxy, phénoxy, alkylthio, halogéno, amido, alkylamido, nitrile, acyle ou nitro.

2. Oxime suivant la revendication 1, dans laquelle $R_1$ et $R_2$ peuvent être égaux ou différents et représentent individuellement un groupe alkyle en $C_1$ à $C_6$.

3. Oxime suivant la revendication 2, dans laquelle $R_1$ et $R_2$ sont des groupes méthyle.

4. Oxime suivant l'une quelconque des revendications 1 à 3, dans laquelle *n* est égal à 0.

5. Oxime suivant l'une quelconque des revendications précédentes, dans laquelle A est un groupe alkylène ou alcénylène acyclique, cyclique ou bicyclique ou un groupe phénylène substitué en ortho qui forme un noyau hétérocyclique pentagonal, hexagonal ou heptagonal insaturé qui peut aussi contenir de l'azote ou un groupe carbonyle en plus des deux atomes d'azote, lesdits systèmes alkylène, alcénylène ou phénylène cycliques étant éventuellement substitués avec un ou plusieurs groupes alkyle en $C_1$ à $C_4$ ou cyclohexyle.

6. Oxime suivant la revendication 5, dans laquelle A forme un noyau de pyrimidine, de diazépine, de triazole, d'imidazole ou de benzimidazole.

7. Le 2,2-diméthyl-3-hydroxyimino-2,3,4,6-tétrahydroimidazo[2,1-b]thiazole.

8. La 2,2-diméthyl-3-hydroxyimino-2,3,4,6-tétrahydro-7H-thiazolo[3,2-a]pyrimidine.

9. La 2,2,5,5,8,8-hexaméthyl-3-hydroxyimino-2,3,5,6,7,8-hexahydrothiazolo[3,2-a][1,3]diazépine.

10. Le 6,6-diméthyl-5-hydroxyimino-5,6-dihydrothiazolo[2,3-c]-1,2,4-triazole.

11. Le 2,2-diméthyl-3-hydroxyimino-2,3-dihydroimidazo[2,1-b]thiazole.

12. Procédé de production d'une oxime de 2-iminothiazolidine bicyclique suivant la revendication 1, procédé qui consiste à faire réagir un composé de formule

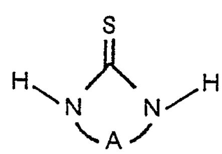

avec un composé de formule

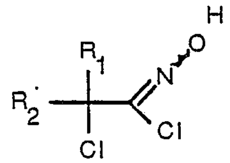

en présence d'un accepteur d'acide, formules dans lesquelles $R_1$, $R_2$ et A ont les définitions données dans la revendication 1.

**Patentansprüche**

1. Ein bicyclisches 2-Iminothiazolidinoxim der allgemeinen Formel

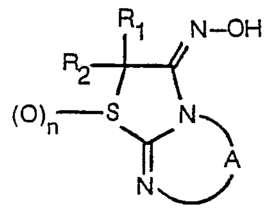

in welcher $R_1$ und $R_2$ gleich oder verschieden sind und einzeln Wasserstoff, $C_1$—$C_6$ Alkyl oder Halogen bedeuten, n = 0, 1 oder 2 ist und A eine acyclische, cyclische oder bicyclische Alkylen- oder Alkenylengruppe oder eine o-substituierte Phenylengruppe ist, die einen ungesättigten 5-, 6- oder 7-gliedrigen heterocylischen Ring bildet, der noch Sauerstoff, Schwefel, Stickstoff oder Carbonyl zusätzlich zu den beiden Stickstoffatomen enthalten kann, wobei die Alkylen, Alkenylen- oder Phenylenringsysteme wahlweise mit einer oder mehreren $C_1$—$C_4$ Alkyl-, Cyclohexyl-, Alkoxy-, Phenoxy-, Alkylthio-, Halogen-, Amido-, Alkylamido-, Nitril-, Acyl- oder Nitrogruppen substituiert sein können.

2. Ein Oxim wie in Anspruch 1, in welchem $R_1$ und $R_2$ gleich oder verschieden sein können und einzeln für $C_1$—$C_6$ Alkyl stehen.

3. Ein Oxim wie in Anspruch 2, in welchem $R_1$ und $R_2$ Methyl sind.

4. Ein Oxim wie in einem der Ansprüche 1 bis 3, in welchem n = 0 ist.

5. Ein Oxim wie in einem der vorhergehenden Ansprüche, in welchem A eine acyclische, cyclische oder bicyclische Alkylen- oder Alkenylengruppe oder eine o-substituierte Phenylengruppe ist, die einen ungesättigten 5-, 6- oder 7-gliedrigen heterocyclischen Ring bildet, der noch Stickstoff oder Carbonyl zusätzlich zu den beiden Stickstoffatomen enthalten kann, wobei die Alkylen-, Alkenylen- oder Phenylenringsysteme wahlweise mit einer oder mehreren $C_1$—$C_4$ Alkyl- oder Cyclohexylgruppen substituiert sind.

6. Ein Oxim wie in Anspruch 5, in welchem A ein Pyrimidin, Diazepin-, Triazol-, Imidazol- oder Benzimidazolringsystem bildet.

7. 2,2-Dimethyl-3-hydroxyimino-2,3,4,6-tetrahydroimidazo[2,1-b]thiazol.

8. 2,2-Dimethyl-3-hydroxyimino-2,3,4,6-tetrahydro-7H-thiazol[3,2-a]pyrimidin.

9. 2,2,5,5,8,8-Hexamethyl-3-hydroxyimino-2,3,5,6,7,8-hexahydrothiazolo[3,2-a][1,3]diazepin.

10. 6,6-Dimethyl-5-hydroxyimino-5,6-dihydrothiazolo[2,3-c]1,2,4-triazol.

11. 2,2-Dimethyl-3-hydroxyimino-2,3-dihydroimidazo[2,1-b]thiazol.

12. Ein Verfahren zur Herstellung eines bicyclischen 2-Iminothiazolidinoxims wie in Anspruch 1, dadurch gekennzeichnet, daß man eine Verbindung der Formel

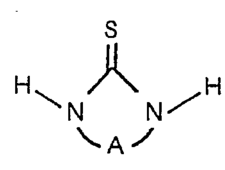

mit einer Verbindung der Formel

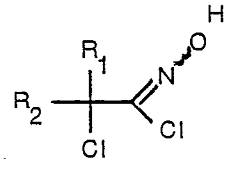

in Anwesenheit eines Säureakzeptors umsetzt, worin $R_1$, $R_2$, n und A wie in Anspruch 1 definiert sind.